# EUROPEAN PATENT SPECIFICATION

(11) **EP 4 431 125 B1**
(45) Date of publication and mention of the grant of the patent: **11.11.2026**
(21) Application number: 24180604.1
(22) Date of filing: 04.05.2018
(51) Int. Cl.: A61L 29/14, A61L 29/08

(54) **LUBRICIOUS HYDROPHILIC COATINGS AND METHODS OF FORMING THE SAME**
LUBRIFIZIERENDE HYDROPHILE BESCHICHTUNGEN UND VERFAHREN ZUR BILDUNG DAVON
REVÊTEMENTS HYDROPHILES LUBRIFIÉS ET LEURS PROCÉDÉS DE FORMATION

(30) Priority: 04.05.2017 US 201762501476 P
(43) Date of publication of application: 18.09.2024
(62) Document number(s) of the earlier application(s) in accordance with Art. 76 EPC: 18726663.0 / 3 618 883
(73) Proprietor: Hollister Incorporated, Libertyville, IL 60048-3781 (US)
(72) Inventor: O'MAHONY, John, P., Libertyville, IL 60048 (US); FARRELL, David, J., Libertyville, IL 60048 (US)
(74) Representative: FRKelly

(56) References cited:
- EP-A1- 0 913 445
- WO-A1-2008/031601
- WO-A1-2010/001813
- WO-A1-2015/066227
- US-A- 5 179 174
- US-A1- 2014 113 070

## Description

### Related Application

The present application claims the benefit of and priority to U.S. Provisional Application No. 62/501,476, filed May 4, 2017.

### Field of the Disclosure

The present disclosure is directed to hydrophilic coatings and methods of forming the same. More particularly, the present disclosure is directed to lubricious hydrophilic coatings formed on the surfaces of medical devices. Even more particularly, the present disclosure is directed to hydrophilic coating formulations that self-crosslink to form a hydrophilic coating when the solvent of the hydrophilic coating formulation is dried off, or otherwise removed, from the formulation.

### Background

It is desirable for medical devices that are inserted into the body to have a lubricated or lubricious outer surface to facilitate insertion into and/or removal of the medical device from the body. Such devices may include, for example, urinary catheters, endoscopes, cardiovascular catheters, syringes, vascular stents, etc. Such medical devices may have a hydrophilic coating or layer disposed on an outer surface thereof. Hydrophilic coatings are becoming the preferred method of providing lubricious surfaces because of their high lubricity and ease of use. Hydrophilic coatings become slippery or lubricious when wetted with a wetting fluid, such as saline or water. The wetted lubricious hydrophilic coating eases insertion and removal of the device, which can result in minimizing soft tissue damage and reducing overall discomfort during use of the medical device.

When a hydrophilically coated medical is used, the hydrophilic coating is typically wetted for a certain period of time prior to use to activate the hydrophilic coating. For example, the user may immerse or otherwise contact the hydrophilic coating with a wetting fluid to wet or activate the coating. In some instances, the medical device is packaged in a packaging that includes liquid or vapor water within the package that hydrates the coating while the device is in the package so that the device is ready to use right out of the package.

Hydrophilic coatings are oftentimes formed on the surfaces of medical devices by a dip coating process that includes dipping the medical device into a hydrophilic coating formulation. The formulation generally includes a solvent, one or more hydrophilic polymers and additives. After the hydrophilic coating formulation has been applied to the surface of the medical device, the coating formulation is cured to form a hydrophilic coating on the surface of the medical device. It is commonplace for the coating formulation to include a photo activated crosslinker and to utilize a radiation or photo curing process to cure the coating formulation. One of the more widely used curing processes is ultraviolet (UV) curing wherein the coating formulation is exposed to UV light, which activates the crosslinker to commence crosslinking of the polymer within the coating formulation to thereby form the hydrophilic coating.

While UV curing is widely used, it is not without its issues. For example, UV curing can require considerable time to achieve the desired degree of crosslinking, which can reduce manufacturing efficiency. Additionally, the materials being crosslinked can be highly sensitive to the UV dose, which can vary as UV lamps degrade over time. This may lead to variations of the level of crosslinking and to under crosslinking of the coating.

Therefore, there remains a need for improved curing/crosslinking processes for forming hydrophilic coatings.

US 5 179 174 describes a crosslinked polyurethane coating formed from a formulation comprising a mixture of isocyanate, a polyol, and a polyethylene oxide in a carrier liquid. A metallic catalyst such as ferric acetylacetonate can be used for the curing reaction, which is initiated upon removal of the solvent and heating.

### Summary

The scope of this invention is defined by the claims. Any "embodiment" or "example" which is disclosed in the description but is not covered by the claims should be considered as presented for illustrative purpose only.

Described herein is a hydrophilically coated medical device that includes a medical device having a surface and a hydrophilic coating disposed on the surface of the medical device. The hydrophilic coating including a hydrophilic polymer and metal.

The present invention provides a formulation for forming such a hydrophilic coating for a medical device. The formulation comprises a hydrophilic polymer in the form of a copolymer comprising a carboxylic acid functionality, a metal acetylacetonate crosslinker, and a solvent that suppresses crosslinking.

Further described herein is a method for forming a hydrophilic coating on a medical device that includes applying a hydrophilic coating formulation as described in the appended claims to a surface of the medical device. The method further includes drying off the solvent of the hydrophilic coating formulation to initiate crosslinking, and thereby forming the hydrophilic coating on the surface of the medical device.

### Detailed Description of the Embodiments

The present disclosure is directed to methods of forming hydrophilic coatings, and more particularly lubricious hydrophilic coatings, on the surface of a substrate, such as a medical device. The methods include applying a hydrophilic coating formulation to the surface of the substrate wherein the hydrophilic coating formulation includes a hydrophilic polymer in the form of a copolymer comprising a carboxylic acid functionality, a metal acetylacetonate crosslinker to crosslink the hydrophilic polymer and a blocking solvent, which may be a volatile stabilizer or may include a volatile stabilizer, that suppresses or blocks the crosslinking reaction. In one embodiment, the solvent may be a single solvent that is a blocking solvent or volatile stabilizer. In another embodiment, the solvent may include 30 wt% or more of a blocking or volatile stabilizing solvent and the remainder a solution, reaction or carrier solvent. After the hydrophilic coating formulation is applied to the surface of the medical device, the blocking solvent is driven, dried off (e.g. evaporated) or otherwise removed. With the solvent driven off, the crosslinker is active and free to start crosslinking reactions, thereby curing the hydrophilic coating formulation and forming the crosslinked hydrophilic coating. The present disclosure is also directed to the hydrophilic coatings formed from such methods and the hydrophilic coating formulations that are employed to form the hydrophilic coatings on a medical device. These hydrophilic coating formulations constitute the claimed subject-matter.

As used herein, the term "hydrophilic coating" means a coating disposed on a surface of a substrate wherein the coating has hydrophilic properties, and preferably becomes highly lubricious when wetted with a wetting fluid. The terms "hydrophilic coating formulation" and "coating formulation" refer to a solution or dispersion that includes the components for forming a hydrophilic coating and that is applied to the surface of a substrate and is dried to form a hydrophilic coating.

The methods of forming hydrophilic coatings of the present disclosure may include applying a hydrophilic coating formulation directly on the surface of a substrate, such as a medical device, and then drying the hydrophilic coating formulation to crosslink the hydrophilic polymer and form a hydrophilic coating on the surface of the substrate. In other embodiments, the methods may include the use of a base coat formulation and a top coat formation wherein a base coat formulation is applied to the surface of a substrate to form a base coat on the surface of the substrate, and then a top coat formulation is applied over the base formulation to form the hydrophilic coating. The base coat may have suitable adhesion/attraction to both the surface of the substrate and the top coat such that the base coat serves as a tie-layer or binding layer.

The hydrophilic coating formulations (including the base coat and top coat formulations) disclosed herein, the hydrophilic coatings formed therefrom and the methods of forming such hydrophilic coatings are particularly useful in the field of urinary catheters. However, the formulations, coatings and methods may be used to coat virtually any medical device for which it is desired to provide a hydrophilic coating on the surface thereof. The formulations, coatings and methods are particularly useful for medical devices that are intended to be inserted into and removed from the body, such as urinary catheters, endoscopes, drainage catheters, etc.

When the methods include the use of base coat formulations and top coat formulations to form the hydrophilic coating, the base and top coat formulations disclosed herein may be used with one another to form a lubricious hydrophilic coating on a substrate. While the base coat and top coat formulations may be used with each other to form hydrophilic coatings, such base and top coat formulations are not required to be used with each other. That is, the base coat formulations disclosed herein may not only be used with the top coat formulations disclosed herein but may also be used with other suitable top coat formulations to form a hydrophilic coating on a medical device. Similarly, the top coat formulations disclosed herein may not only be used with the base coat formulations disclosed herein but may also be used with other suitable base coat formulations to form a hydrophilic coating on the surface of a medical device. Furthermore, as mentioned above the top coat formulations or hydrophilic coating formulations may be applied directly to the surface of the substrate to form a hydrophilic coating on the substrate without the use of a base coat.

The hydrophilic coating formulations according to the claimed invention (including base and top coat formulations) include a hydrophilic polymer in the form of a copolymer comprising a carboxylic acid functionality, a metal acetylacetonate crosslinker and a blocking solvent that suppresses crosslinking. The hydrophilic polymer may be a copolymer of a hydrophilic polymer and an acid group such as carboxylic acid. The carboxylic acid may include but is not limited to acrylic acid and methacrylic acid. The hydrophilic polymer may include but is not limited to polyvinylpyrrolidone (PVP) or polyethylene oxide (POE).

The metal chelating crosslinker is a metal-acetylacetonate crosslinker, preferably wherein the metal is titanium, aluminum, zinc, or any other metal suitable for medical use. For example, the crosslinker may have formula of:

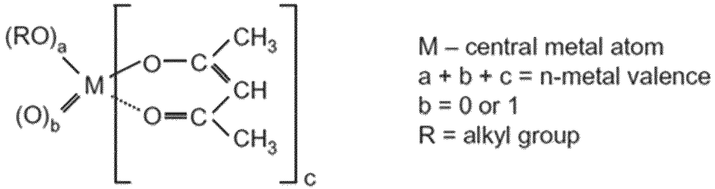

The solvent is a crosslink suppressing, blocking or volatile stabilizing solvent that renders the crosslinking reaction between the hydrophilic polymer and the metal chelating crosslinker inactive. The solvent may be such a blocking solvent or the solvent may be a mixture of solvents wherein one of the solvents is such a blocking solvent. Such blocking solvents may include 2, 4 pentanedione or isopropyl alcohol. Accordingly, the solvent may be a mixture that includes 2, 4 pentanedione and/or isopropyl alcohol, along with another solvent. The formulation also may include additives, such as plasticizer, osmolality increase agent and antioxidant.

In one exemplary hydrophilic coating formulation, the formulation may include the solvent in an amount of between about 50 weight percent (wt%) and about 98 wt%, hydrophilic polymer in an amount between about 1.95 wt% and about 48.5 wt%, and a metal chelating crosslinker in amount between about 0.05 wt% and about 1.5 wt%. In one embodiment, the formulation includes isopropyl alcohol as the solvent and volatile stabilizer, poly (N-vinylpyrrolidone-co-acrylic acid) as the hydrophilic polymer and Titanium acetylacetonate (TiACA) as the metal chelating crosslinker, for example Ti(C₅H₇O₂)₂(C₃H₇O)₂.

In one method of forming a hydrophilic coating, a hydrophilic coating formulation as defined in the appended claims is applied directly to a surface of a substrate. The hydrophilic coating formulation may be applied by, for example, dip coating, spraying or brushing. After the hydrophilic coating formulation has been applied to the surface, the blocking solvent is dried off leaving the metal chelating crosslinker reactive and free to commence crosslinking reactions to crosslink the hydrophilic polymer, thereby crosslinking the polymer and forming a hydrophilic coating on the surface of the substrate. The blocking solvent may be dried or driven off by heating the hydrophilic coating formulation. In some embodiments, the crosslinking process will continue even after the heat is removed and while the solvent continues to dry off of the hydrophilic coating formulation/hydrophilic coating.

In another method of forming a hydrophilic coating, a base hydrophilic coating formulation as defined in the appended claims is applied to the surface of the substrate and the blocking solvent is dried off to crosslink the hydrophilic polymer, thereby forming a base coat. A top hydrophilic coating formulation as defined in the appended claims is applied over the base coat and the blocking solvent is dried off to crosslink the hydrophilic polymer of the top coat formulation, thereby forming a hydrophilic coating.

The hydrophilic coatings disclosed herein are preferably coatings that become lubricious when wetted with a wetting agent, such as water. The resulting hydrophilic coating includes a crosslinked hydrophilic polymer, a metal (of the metal chelating crosslinker) and, optionally, additives. The metal is bonded to the hydrophilic polymer which may aid in reducing the level of leachables from the hydrophilic coating.

There are advantages that may be obtained from the methods, formulations and coatings disclosed herein. For example, the need for radiation/photo curing may be reduced or eliminated, which may result in shorter and more efficient manufacturing times. The level of crosslinking and the ability to reproduce consistency in the level of crosslinking can be improved through varying the stoichiometric ratios of the hydrophilic polymer and the crosslinking agent.

## Claims

1. A formulation for forming a hydrophilic coating for a medical device, comprising:
a hydrophilic polymer in the form of a copolymer comprising a carboxylic acid functionality;
a metal acetylacetonate crosslinker; and
a solvent that suppresses crosslinking.

2. The formulation of claim 1 wherein the solvent comprises 2,4 pentanedione or isopropyl alcohol.

3. The formulation of any one of claims 1-2 wherein the metal acetylacetonate crosslinker comprises one or more of titanium, aluminum, and zinc,

4. The formulation of any one of claims 1-3 wherein the hydrophilic polymer comprises polyethylene oxide.

5. The formulation of any one of claims 1-3 wherein the hydrophilic polymer comprises polyvinylpyrrolidone.

6. The formulation of claim 1-5 wherein the carboxylic acid is acrylic acid or methacrylic acid.

7. The formulation of any one of claims 1-6 wherein the hydrophilic polymer comprises a copolymer comprising a monomer having a functional hydroxyl group.

8. The formulation of claim 7 wherein the monomer comprises hydroxyethylacrylate and/or hydroxyethylmethacrylate.

9. The formulation of any one of claims 1-8 further comprising one or more of plasticizer, osmolality increase agent and antioxidant.

## Patentansprüche

1. Formulierung zur Bildung einer hydrophilen Beschichtung für eine medizinische Vorrichtung, umfassend:
ein hydrophiles Polymer in der Form eines Copolymers, das eine Carbonsäurefunktionalität umfasst;
einen Metallacetylacetonat-Vernetzer; und
ein Lösungsmittel, das ein Vernetzen unterdrückt.

2. Formulierung nach Anspruch 1, wobei das Lösungsmittel 2,4 Pentandion oder Isopropylalkohol umfasst.

3. Formulierung nach einem der Ansprüche 1-2, wobei der Metallacetylacetonat-Vernetzer eines oder mehrere von Titan, Aluminium und Zink umfasst.

4. Formulierung nach einem der Ansprüche 1-3, wobei das hydrophile Polymer Polyethylenoxid umfasst.

5. Formulierung nach einem der Ansprüche 1-3, wobei das hydrophile Polymer Polyvinylpyrrolidon umfasst.

6. Formulierung nach Anspruch 1-5, wobei die Carbonsäure Acrylsäure oder Methacrylsäure ist.

7. Formulierung nach einem der Ansprüche 1-6, wobei das hydrophile Polymer ein Copolymer umfasst, das ein Monomer umfasst, das eine funktionelle Hydroxylgruppe aufweist.

8. Formulierung nach Anspruch 7, wobei das Monomer Hydroxyethylacrylat und/oder Hydroxyethylmethacrylat umfasst.

9. Formulierung nach einem der Ansprüche 1-8, ferner umfassend eines oder mehrere von einem Weichmacher, einem Osmolalitätserhöhungsmittel und einem Antioxidans.

## Revendications

1. Formulation pour former un revêtement hydrophile pour un dispositif médical, comprenant :
un polymère hydrophile sous la forme d'un copolymère comprenant une fonctionnalité acide carboxylique ;
un agent de réticulation à base d'acétylacétonate de métal ; et
un solvant qui inhibe la réticulation.

2. Formulation selon la revendication 1, dans laquelle le solvant comprend de la 2,4-pentanedione ou de l'alcool isopropylique.

3. Formulation selon l'une quelconque des revendications 1 à 2, dans laquelle l'agent de réticulation à base d'acétylacétonate de métal comprend l'un ou plusieurs parmi le titane, l'aluminium et le zinc.

4. Formulation selon l'une quelconque des revendications 1 à 3, dans laquelle le polymère hydrophile comprend du poly(oxyde d'éthylène).

5. Formulation selon l'une quelconque des revendications 1 à 3, dans laquelle le polymère hydrophile comprend de la polyvinylpyrrolidone.

6. Formulation selon l'une quelconque des revendications 1 à 5, dans laquelle l'acide carboxylique est l'acide acrylique ou l'acide méthacrylique.

7. Formulation selon l'une quelconque des revendications 1 à 6, dans laquelle le polymère hydrophile comprend un copolymère comprenant un monomère ayant un groupe hydroxyle fonctionnel.

8. Formulation selon la revendication 7, dans laquelle le monomère comprend de l'acrylate d'hydroxyéthyle et/ou du méthacrylate d'hydroxyéthyle.

9. Formulation selon l'une quelconque des revendications 1 à 8, comprenant en outre l'un ou plusieurs parmi un plastifiant, un agent augmentant l'osmolalité et un antioxydant.
